# EUROPEAN PATENT SPECIFICATION

(11) **EP 4 187 228 B1**
(45) Date of publication and mention of the grant of the patent: **16.10.2024**
(21) Application number: 22208866.8
(22) Date of filing: 22.11.2022
(51) Int. Cl.: G01N 15/05, G01N 33/49, G01N 15/02, G01N 15/14, G06N 3/02, G01N 15/12, G01N 15/01, G01N 15/00

(54) **SYSTEM, APPARATUS, AND METHOD FOR MEASURING ERYTHROCYTE SEDIMENTATION RATE**
SYSTEM, VORRICHTUNG UND VERFAHREN ZUR MESSUNG DER ERYTHROZYTENSEDIMENTATIONSRATE
SYSTÈME, APPAREIL ET PROCÉDÉ DE MESURE DE LA VITESSE DE SÉDIMENTATION DES ÉRYTHROCYTES

(30) Priority: 25.11.2021 CN 202111414435
(43) Date of publication of application: 31.05.2023
(73) Proprietor: Shenzhen Mindray Bio-Medical Electronics Co., Ltd., Shenzhen, Guangdong 518057 (CN)
(72) Inventor: ZHENG, Wenbo, Shenzhen, 518057 (CN); SI, Jinxiu, Shenzhen, 518057 (CN); YE, Bo, Shenzhen, 518057 (CN); YE, Yi, Shenzhen, 518057 (CN); QI, Huan, Shenzhen, 518057 (CN)
(74) Representative: KIPA AB

(56) References cited:
- EP-A1- 4 063 825
- WO-A1-2021/097610

## Description

### TECHNICAL FIELD

The disclosure relates to the field of in vitro diagnostics, and in particular, to an apparatus, a system, and a method for measuring erythrocyte sedimentation rate.

### BACKGROUND

Erythrocyte sedimentation rate (ESR for short) refers to a rate at which red blood cells in anticoagulated blood in vitro naturally sediment under specified conditions. The erythrocyte sedimentation rate is used for the differential diagnosis and observation for some diseases, and it is also a common indicator that reflects aggregation of red blood cells. The ESR is of significance for discerning a quiescent stage of a disease, stability and relapse of conditions, and benignancy and malignancy of tumors.

Currently, the ESR is usually measured by using a Westergren method in the art. That is, a sedimentation rate of red blood cells in a blood sample contained in a Westergren tube or an ESR tube is observed and recorded. Usually, in the Westergren method, after injection of the blood sample into the Westergren tube, timer is started, and a distance from an interface between clustered cells and a plasma to a top liquid level at the top of the tube (a height of the suspending medium) is observed in one hour.

However, measuring the ESR by using the Westergren method (also referred to as a reference method) has the following defects. 1) A measurement speed thereof is slow. Sedimentation time of one hour is required. However, with the increase of the number of patients in a hospital, the testing efficiency of providing measurement results in one hour cannot meet the daily testing needs. 2) More blood volume is needed for this method. Usually, a blood volume of about 1 mL is needed to fill up the ESR tube, which is impossible for patients whose peripheral finger blood is collected to be tested.

In order to overcome the above defects of the Westergren method, a fast ESR measurement method (also referred to as an erythrocyte aggregation method hereinafter) has emerged in the art on the basis of hemorheology research, which is a method for predicting erythrocyte sedimentation rate by measuring an erythrocyte aggregation index. A measured value of the ESR may be derived by measuring a change in a scattering rate/transmissivity of blood cells to light during the formation of rouleaux red blood cells. This measurement process can be completed within a very short time (about 20 s), and the blood volume used for it is only about 100 uL. These methods show the erythrocyte aggregation index is correlated to the erythrocyte sedimentation rate at a certain level.

However, although an ESR value can be rapidly obtained by using the erythrocyte aggregation method, there is still a significant discrepancy between an ESR value measured by using the erythrocyte aggregation method and an ESR value measured by using the Westergren method. Patent application WO 2021/097610A1 provides teachings related to the technical field of the application.

### SUMMARY

Therefore, an object of the disclosure is to provide an apparatus for measuring ESR, a system for measuring ESR, and a method for measuring ESR, which can rapidly and accurately obtain a corrected ESR measurement result. The ESR measurement result obtained by the disclosure has a good consistency with the ESR measurement result obtained by using the Westergren method.

To achieve the object of the disclosure, a first aspect of the disclosure provides an apparatus for measuring erythrocyte sedimentation rate, including an ESR detection device and a data processing device. The ESR detection device is configured to test a blood sample to be tested, to obtain an erythrocyte aggregation curve of light intensity transmitted through the blood sample to be tested or of light scattered by the blood sample to be tested as a function of time. The data processing device is configured to: acquire the erythrocyte aggregation curve of the blood sample to be tested, acquire a blood cell histogram and/or a blood cell scattergram of the blood sample to be tested that are/is obtained by a blood cell analyzer, and input the erythrocyte aggregation curve and the blood cell histogram and/or the blood cell scattergram into a neural network model, to calculate an ESR measurement result by the neural network model.

In the apparatus provided in the first aspect of the disclosure, the erythrocyte aggregation curve of the blood sample to be tested is rapidly obtained by the ESR detection device based on an erythrocyte aggregation method, and then the blood cell histogram and/or the blood cell scattergram of the blood sample to be tested are/is obtained from the blood cell analyzer which is outside the apparatus for measuring the ESR. Next, the erythrocyte aggregation curve and the blood cell histogram and/or the blood cell scattergram are input into the neural network model, especially a pre-trained neural network model. In this way, an accurate ESR measurement result can be output.

A second aspect of the disclosure provides a system for measuring erythrocyte sedimentation rate, including a sampling and dispensing device, a blood cell detection device, and the apparatus for measuring ESR in the first aspect. The sampling and dispensing device is configured to collect a blood sample to be tested, and dispense at least portions of the blood sample to be tested into the ESR detection device and the blood cell detection device respectively. The ESR detection device is configured to test the portion of the blood sample dispensed therein, so as to obtain the erythrocyte aggregation curve of the light intensity transmitted through the portion of the blood sample dispensed therein or of light scattered by the portion of the blood sample dispensed therein as a function of time. The blood cell detection device is configured to test the portion of the blood sample dispensed therein to obtain the blood cell histogram and/or the blood cell scattergram of the blood sample to be tested. The blood cell histogram and/or the blood cell scattergram include/includes at least a histogram and/or a scattergram related to red blood cells. The data processing device is configured to acquire the erythrocyte aggregation curve and the blood cell histogram and/or the blood cell scattergram, and input the erythrocyte aggregation curve and the blood cell histogram and/or the blood cell scattergram into the neural network model, so as to calculate a first ESR measurement result by using the neural network model.

In the system provided in the second aspect of the disclosure, by integrating the ESR detection device and the blood cell detection device, the erythrocyte aggregation curve and the red blood cell histogram and/or the red blood cell scattergram of the blood sample to be tested can be simultaneously acquired rapidly, and the erythrocyte aggregation curve and the blood cell histogram and/or the blood cell scattergram are input into the neural network model, especially a pre-trained neural network model, so as to output an accurate ESR measurement result.

A third aspect of the disclosure provides a method for measuring erythrocyte sedimentation rate, including the following operations.

An erythrocyte aggregation curve of a blood sample to be tested is acquired.

A blood cell histogram and/or a blood cell scattergram of the blood sample to be tested are/is acquired, in which the blood cell histogram and/or the blood cell scattergram include/includes at least a histogram and/or a scattergram related to red blood cells.

The erythrocyte aggregation curve and the blood cell histogram and/or the blood cell scattergram are input into a neural network model, to calculate a first ESR measurement result by using the neural network model.

In the method provided in the third aspect of the disclosure, the acquired erythrocyte aggregation curve and the acquired blood cell histogram and/or blood cell scattergram are input into the neural network model, especially a pre-trained neural network model. In this way, an accurate ESR measurement result can be output.

### BRIEF DESCRIPTION OF THE DRAWINGS

The disclosure will be more clearly elaborated below with reference to the examples and the accompanying drawings. The above and other advantages will become apparent to those of ordinary skill in the art from the detailed description of the examples of the disclosure. The accompanying drawings are only used for illustrating preferred examples and should not be construed as limitations to the disclosure. The same or similar reference numerals represent the same components throughout the accompanying drawings. In the accompanying drawings:
FIG. 1 is a schematic block diagram of an example of an apparatus for measuring ESR according to the disclosure;
FIG. 2 is a schematic structural diagram of an example of an ESR detection device of the apparatus for measuring ESR in FIG. 1;
FIG. 3 is a schematic structural diagram of an example of a neural network model according to the disclosure;
FIG. 4 is a schematic block diagram of an example of a system for measuring ESR according to the disclosure;
FIG. 5 is a schematic structural diagram of an example of a system for measuring ESR according to the disclosure;
FIG. 6 is a schematic structural diagram of an example of an impedance detection unit of a blood cell detection device according to the disclosure;
FIG. 7 is a schematic structural diagram of an example of an optical detection unit of a blood cell detection device according to the disclosure;
FIG. 8 shows an erythrocyte aggregation curve obtained by an ESR detection device according to the disclosure by using an erythrocyte aggregation method;
FIG. 9 shows a calibration curve for calculating an ESR measurement result before correction;
FIG. 10 shows a correlation between an ESR measurement result before correction that is measured according to the disclosure and an ESR measurement result that is measured by using an erythrocyte aggregation method in the art;
FIG. 11 is a red blood cell volume distribution histogram obtained by the impedance detection unit shown in FIG. 6;
FIG. 12 shows a correlation between an ESR measurement result corrected by using the red blood cell volume distribution histogram shown in FIG. 11 according to the disclosure and a measurement result obtained by the Westergren method;
FIG. 13 is a red blood cell scattergram obtained by the impedance detection unit shown in FIG. 6;
FIG. 14 shows a correlation between an ESR measurement result corrected by using the red blood cell scattergram shown in FIG. 13 according to the disclosure and a measurement result obtained by the Westergren method;
FIG. 15 is a red blood cell scattergram obtained by the optical detection unit shown in FIG. 7;
FIG. 16 is a schematic flowchart of an example of a method for measuring ESR according to the disclosure; and
FIG. 17 is a schematic flowchart of another example of a method for measuring ESR according to the disclosure.

### DETAILED DESCRIPTION

The examples of the disclosure will be clearly and fully described below with reference to the accompanying drawings. Apparently, the examples to be described are merely some, rather than all, of the examples of the disclosure.

It should be noted that the term "first/second/third" in the examples of the disclosure is only used to distinguish similar objects, and does not represent specific order of the objects. It may be understood that the specific order or sequence of "first/second/third" may be interchanged if applicable.

It can be understood by those skilled in the art that all terms including technical terms and scientific terms used herein have the same meanings as those generally understood by those of ordinary skill in the art to which the present application pertains, unless otherwise specified.

Currently, an ESR value of a blood sample is increasingly used clinically to assist in diagnosis of diseases. The inventors of the disclosure have noticed that consistency between an ESR value obtained by an existing erythrocyte aggregation method and an ESR value obtained by the reference method (that is, the Westergren method) is poor. In other words, the ESR value obtained by the erythrocyte aggregation method cannot accurately reflect a natural sedimentation rate of red blood cells.

In order to rapidly obtain an ESR value that can more accurately reflect the natural sedimentation rate of red blood cells, a technical solution is proposed to correct an erythrocyte aggregation curve by using a blood cell distribution diagram obtained by a blood cell analyzer.

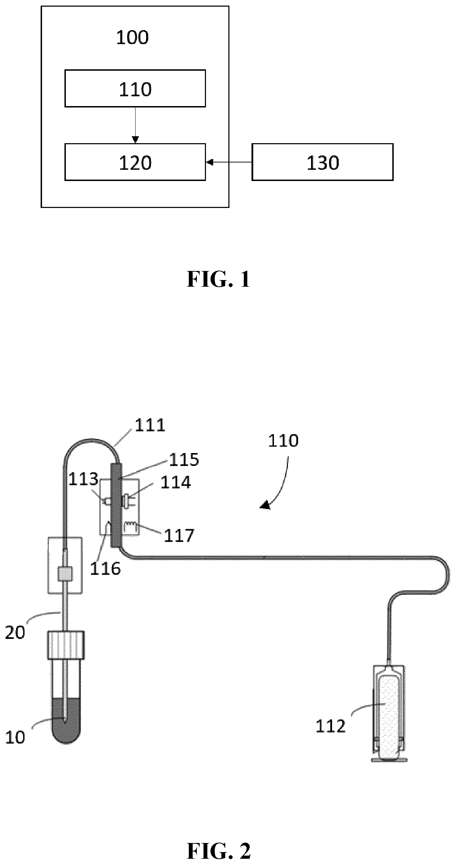

As shown in FIG. 1, according to an example of the disclosure, an apparatus 100 for measuring ESR is proposed. Herein, the apparatus 100 for measuring ESR includes an ESR detection device 110 and a data processing device 120.

The ESR detection device 110 is configured to test a blood sample to be tested, to obtain an erythrocyte aggregation curve P1 of light intensity transmitted through a dispensed blood sample portion or scattered by the blood sample portion as a function of time. That is, the ESR detection device 110 is configured to test the blood sample to be tested based on the erythrocyte aggregation method.

The data processing device 120 is configured to process and calculate data to obtain a desired result. In the example of the disclosure, the data processing device 120 is configured to: acquire the erythrocyte aggregation curve P1; acquire a blood cell histogram and/or a blood cell scattergram P2 of the blood sample to be tested that are/is obtained by a blood cell analyzer 130; and input the erythrocyte aggregation curve P1 and the blood cell histogram and/or the blood cell scattergram P2 into a neural network model M, so as to calculate an ESR measurement result ESR1 by the neural network model M. Here, the blood cell histogram and/or the blood cell scattergram include/includes at least a histogram and/or a scattergram related to red blood cells.

Here, the neural network model M includes a deep learning algorithm having a neural network structure. That is, the data processing device 120 is configured to input the analysis data P1 and P2 into the deep learning algorithm having the neural network structure, and calculate the ESR measurement result ESR1 of the blood sample to be tested by means of the deep learning algorithm.

In the example shown in FIG. 1, the blood cell analyzer 130 is arranged outside the apparatus 100 for measuring ESR and is connected to the data processing device 120 in communication so that the data processing device 120 acquires the blood cell histogram and/or the blood cell scattergram obtained by the blood cell analyzer 130.

In an example of the ESR detection device 110, as shown in FIG. 2, the ESR detection device 110 includes a testing pipeline 111, a power assembly 112, and an optical detection assembly with a light emitter 113 and a light receiver 114. The power assembly 112 (e.g., a syringe here) is configured to transfer, for example, aspirate, the blood sample to be tested into a testing region 115 of the testing pipeline 111. For example, the power assembly is configured to aspirate the blood sample to be tested from a test tube 10 to the testing region 115 of the testing pipeline 111 by a sampling needle 20. The light emitter 113 and the light receiver 114 are respectively located on either side of the testing region 115 of the testing pipeline 11. The light emitter 113 is configured to irradiate the blood sample in the testing region 115. The light receiver 114 is configured to detect a change in the amount of light emitted by the light emitter 113 after the light passes through the blood sample (for example, to receive light transmitted through and/or scattered by the blood sample), and detect a degree to which the blood sample in the testing region 115 absorbs or scatters light by detecting the amount of the received light. Since the scatterance or transmittance of light passing through the blood sample may change during aggregation of red blood cells in the blood sample (to form rouleaux), it is possible to detect the degree to which the blood sample absorbs or scatters light and thus to measure the erythrocyte sedimentation rate, by detecting the amount of light transmitted through or scattered by the blood sample. Here, the optical detection assembly tests the blood sample, in particular by using transmission turbidimetry.

When the ESR detection device 110 is activated for testing, the power assembly 112 drives the blood sample to be tested to flow into the testing pipeline 111, allows the blood sample to be tested to stop flowing when it flows to the testing region 115, and then keeps the blood sample to be tested still. The light emitter 113 irradiates the blood sample in the testing region, and the light receiver 114 detects the scatterance or transmittance of the light emitted by the light emitter 113 after it passes through the blood sample in the testing region 115, to detect an erythrocyte aggregation degree, such as an erythrocyte aggregation rate, in the blood sample to be tested.

In some examples, the testing pipeline 111 is made of a flexible tube, and the testing region 115 of the testing pipeline 111 is made of a transparent material. Therefore, the testing pipeline 111 may be arranged arbitrarily and flexibly. For example, it may be arranged vertically, horizontally, or obliquely, or may be arranged in a curved manner, which is not limited herein. Preferably, the testing pipeline 111 is configured as a capillary tube.

In some examples, the ESR detection device 110 may be further configured to: before obtaining the erythrocyte aggregation curve P1 by means of testing, de-aggregate red blood cells in the blood sample to be tested by allowing the blood sample to be tested to flow back and forth in the testing region 115 of the ESR detection device 110. As a result, the red blood cells in the blood sample in the testing region 115 are dispersed as much as possible before the optical detection assembly with the light emitter 113 and the light receiver 114 detects the erythrocyte aggregation degree, so that the erythrocyte aggregation degree can be measured more accurately.

To this end, in the example shown in FIG. 2, the power assembly 112 can be used to drive the blood sample in the testing region 115 of the testing pipeline 11 to flow back and forth. Especially, when the power assembly 112 is configured as a syringe, the red blood cells in blood sample can be flexibly de-aggregated, and a blood volume can be saved, since a movement speed and a movement direction of the syringe can be flexibly set.

After the power assembly 112 drives the blood sample in the testing region 115 to flow back and forth a predetermined number of cycles, the power assembly 112 immediately stops driving, so that the blood sample in the testing region 115 stops flowing, and red blood cells in the blood sample in the testing region 115 aggregate, resulting in the change in transmittance.

In addition, in the example shown in FIG. 2, the ESR detection device 110 may further include a temperature sensor 116 and a heater 117 arranged near the testing region 115 of the testing pipeline 111. The temperature sensor 116 is configured to detect a temperature value of the testing region 115 or of the blood sample in the testing region. The heater 117 is configured to heat the testing region 115 when the temperature value is less than a predetermined temperature to maintain the temperature of the testing region 115 constant.

In some examples, the data processing device 120 may include a processor. The processor may include, but is not limited to, a central processing unit (CPU), a microcontroller unit (MCU), a field-programmable gate array (FPGA), a digital signal processor (DSP) and other devices for interpreting computer instructions and processing data in computer software.

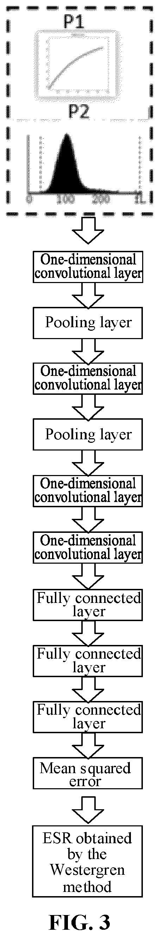

FIG. 3 is a schematic block diagram of an example of a neural network model M according to the disclosure. The neural network model M has a convolutional neural network structure. The convolutional neural network structure includes a data input layer, a convolutional layer, a pooling layer, and a fully connected layer. In the example shown in FIG. 3, the neural network model M includes four convolutional layers, two pooling layers, and three fully connected layers. In other examples, the neural network model M may alternatively have fewer or more convolutional layers, pooling layers, and fully connected layers, which are not specifically limited in the disclosure.

Here, preferably, the neural network model M is pre-trained and stored in the data processing device 120. For example, the neural network model M is pre-trained with testing data of a large number of clinical samples.

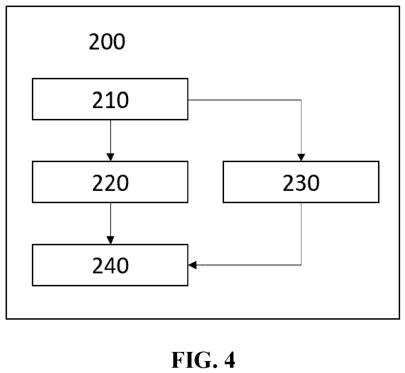

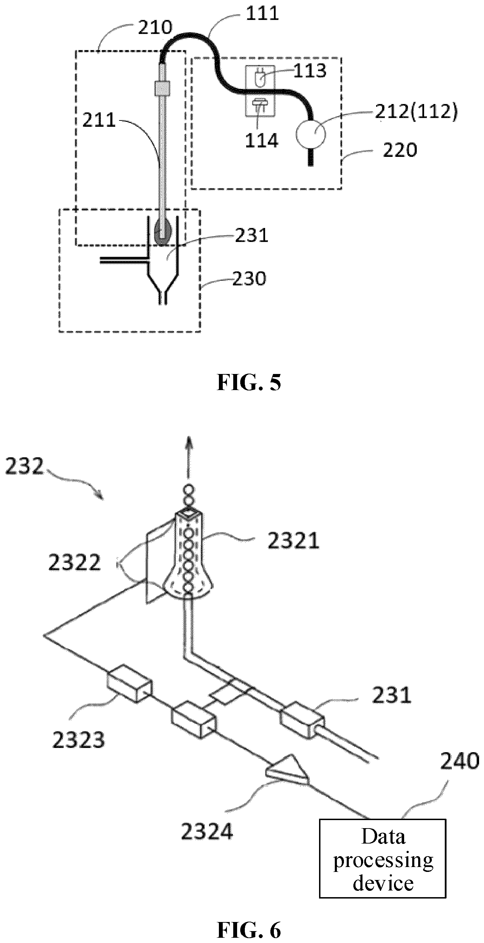

As shown in FIG. 4 and FIG.5, according to another example of the disclosure, a system 200 for measuring ESR is proposed, in which the system integrates an apparatus for measuring ESR and a hematology analyzer. The system 200 includes a sampling and dispensing device 210, an ESR detection device 220, a blood cell detection device (the hematology analyzer) 230, and a data processing device 240.

The sampling and dispensing device 210 is configured to collect a blood sample to be tested, and dispense at least portions of the blood sample to be tested into the ESR detection device 220 and the blood cell detection device 230.

The ESR detection device 220 is configured to test the portion of the blood sample dispensed therein, so as to obtain an erythrocyte aggregation curve P1 of light intensity transmitted through the blood sample to be tested or light scattered by the blood sample to be tested as a function of time. For a structure of the ESR detection device 220, reference may be made to the above description of the ESR detection device 110 shown in FIG. 2, and details will not be described herein again.

The blood cell detection device 230 is configured to test the portion of the blood sample dispensed therein, so as to obtain a blood cell histogram and/or a blood cell scattergram of the blood sample to be tested. Herein, the blood cell histogram and/or the blood cell scattergram include/includes at least a histogram and/or a scattergram related to red blood cells.

The data processing device 240 is configured to acquire the erythrocyte aggregation curve P1 and the blood cell histogram and/or the blood cell scattergram P2, and input the erythrocyte aggregation curve P1 and the blood cell histogram and/or the blood cell scattergram P2 into a neural network model M, to calculate a first ESR measurement result ESR1 by using the neural network model M, that is, an ESR measurement result corrected by using the blood cell histogram and/or the blood cell scattergram. For a structure of the data processing device 240, reference may be made to the above description of the data processing device 120 shown in FIG. 1, and details will not be described herein again.

As shown in FIG. 5, in some examples, the sampling and dispensing device 210 may include a sampling needle 211 and a drive assembly (not shown in the figure). The drive assembly is configured to drive the sampling needle 211 to move, so that the sampling needle 211 collects the blood sample and dispenses a portion of the blood sample into a reaction cell 231 for routine blood test of the blood cell detection device 230. In addition, the sampling and dispensing device 210 may include a power assembly 212, such as a syringe, for providing driving force to aspirate the blood sample from a test tube to a sampling needle.

In some preferred examples, the power assembly of the ESR detection device and the power assembly 212 of the sampling and dispensing device 210 are the same assembly. That is, the power assembly 212 of the sampling and dispensing device 210 can also be used as the power assembly of the ESR detection device.

In some examples, the blood cell detection device 230 includes a reaction cell 231 and a detection assembly (not shown in FIG. 5). The reaction cell 231 is configured to provide a place for the dispensed blood sample to mix with a reagent. The detection assembly is configured to perform a routine blood test on a sample solution to be tested obtained by mixing the blood sample with the reagent in the reaction cell 231.

Those skilled in the art can understand that the detection assembly of the blood cell detection device 230 may include at least one of an optical detection unit, an impedance detection unit, and a hemoglobin detection unit. Accordingly, the reaction cell may include at least one of an optical detection reaction cell, an impedance detection reaction cell, and a hemoglobin detection reaction cell. When the blood cell detection device 230 performs a routine blood test on the blood sample, the blood sample and a corresponding reagent (such as a diluent and/or a hemolyzing agent and/or a staining agent) can be added to the reaction cell 231. The detection assembly can detect the blood sample in the reaction cell 231 to obtain at least one blood cell parameter. Herein, the blood cell parameter may include at least one of or a combination of a white blood cell (WBC) classification parameter, a WBC count and morphological parameter, a hemoglobin (HGB) parameter, a red blood cell (RBC) count and morphological parameter, and a blood platelet (PLT) count and morphological parameter.

In an example of an impedance detection unit 232 of the blood cell detection device 230, as shown in FIG. 6, the impedance detection unit 232 includes a detection aperture 2321 and a detection circuit. The detection circuit is configured to detect a signal of impedance change when cells in the portion of the blood sample dispensed into the impedance detection unit 232 pass through the detection aperture 2321, to obtain a red blood cell parameter and/or a platelet parameter and/or a white blood cell parameter. The impedance detection unit 232 is configured to operate based on a Coulter principle (also known as an electrical impedance method), and in particular, is configured as a sheath flow impedance detection unit. Specifically, the impedance detection unit 232 includes a flow cell having a detection aperture 2321 and includes a detection circuit. The detection circuit includes a pair of electrodes 2322, a direct current (DC) power supply 2323 electrically connected to the electrodes 2322, and an amplifier 2324. The impedance detection unit 232 detects DC impedance generated by particles in the blood sample passing through the detection aperture 2321, and outputs an electrical signal representing information of the particles passing through the aperture. The impedance detection unit 232 may be further provided with a sheath fluid cell (shown in the figure) for supplying a sheath fluid to the flow cell. In the flow cell, the blood sample to be tested flows with being wrapped by the sheath fluid, and particles contained in the blood sample to be tested pass through the detection aperture 2321 one by one. The DC power supply 2323 provides DC power to the pair of electrodes 2322. During the DC power supply provides DC power, impedance between the pair of electrodes 2322 can be detected. A resistance signal representing a change in impedance is amplified by the amplifier 2324 and then transferred to the data processing device 240, to obtain a red blood cell parameter and/or a platelet parameter and/or a white blood cell parameter.

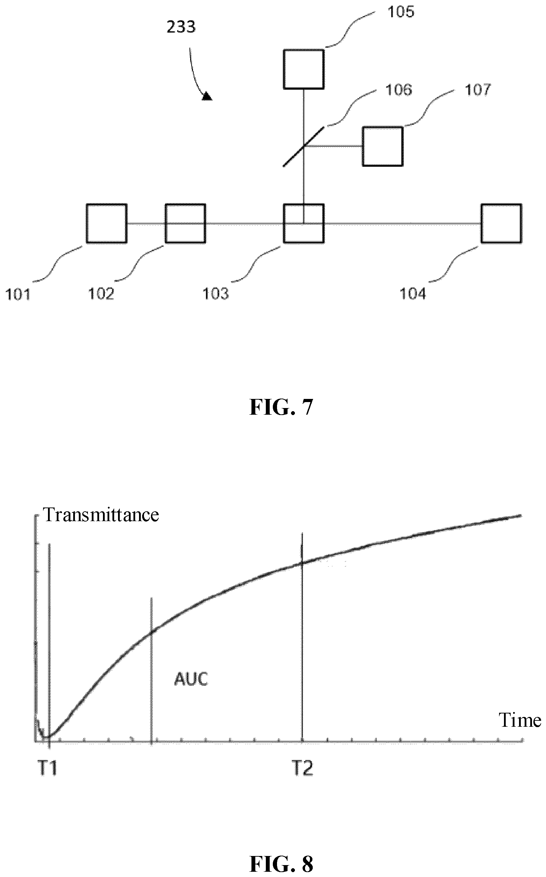

In an example of an optical detection unit 233 of the blood cell detection device 230, as shown in FIG. 7, the optical detection unit 233 is configured to operate based on a principle of flow cytometry, and it has a light source 101, a flow cell 103, and light detectors 104, 105, and 107. The light source 101 is configured to emit a light beam to irradiate a testing region of the flow cell 103, and the light detectors are configured to detect optical signals generated when the cells in the portion of the blood sample dispensed into the optical detection unit 233 pass through the testing region and are irradiated.

Herein, the flow cell 103 refers to a chamber of a focused flow, which is suitable for detecting a scattered light signal and a fluorescence signal. When a particle, such as a blood cell, passes through the detection aperture of the flow cell, the particle scatters an incident light beam to various directions, in which the incident light beam is from the light source and directed to the detection aperture. Light detectors, which may be provided at one or more different angles relative to the incident light beam, may detect light scattered by the particle to obtain a scattered light signal. For example, at least one of a forward-scattered light detector for detecting forward-scattered light (FSC), a side-scattered light detector for detecting side-scattered light (SSC), and a fluorescence detector for detecting fluorescence (SFL) may be provided. The forward-scattered light represents a volume of the cell, the side-scattered light represents internal complexity of the cell, and the fluorescence represents a content of nucleic acid in the cell.

In the example shown in FIG. 7, the optical detection unit 233 has the light source 101, a beam shaping assembly 102, a flow cell 103, and the forward-scattered light detector 104 sequentially arranged in a straight line. On one side of the flow cell 103, a dichroscope 106 is arranged at an angle of 45° relative to the straight line. Part of lateral light caused by particles in the flow cell 103 is transmitted through the dichroscope 106 and is captured by the fluorescence detector 105 arranged behind the dichroscope 106 at an angle of 45° relative to the dichroscope 106; and the other part of the lateral light is reflected by the dichroscope 106 and is captured by the side-scattered light detector 107 arranged in front of the dichroscope 106 at an angle of 45° relative to the dichroscope 106.

In some examples, the erythrocyte aggregation curve P1 is obtained by transmission turbidimetry.

FIG. 8 shows the erythrocyte aggregation curve P1 obtained by the ESR detection devices 110 and 220 according to the disclosure, where transmittance (also referred to as transmissivity) means a relative light intensity, which is equal to a ratio of intensity of transmitted light to intensity of the background light.

In some examples, the data processing devices 120 and 240 may be further configured to: calculate an Area Under Curve (AUC), which refers to an area enclosed by the erythrocyte aggregation curve and a time axis within a time period between a measurement start time point T1 and a measurement end time point T2; and calculate a second ESR measurement result ESR2 (that is, an uncorrected ESR measurement result) based on the AUC and a pre-stored calibration curve.

In some examples, the pre-stored calibration curve is stored, for example, in the data processing devices 120 and 240.

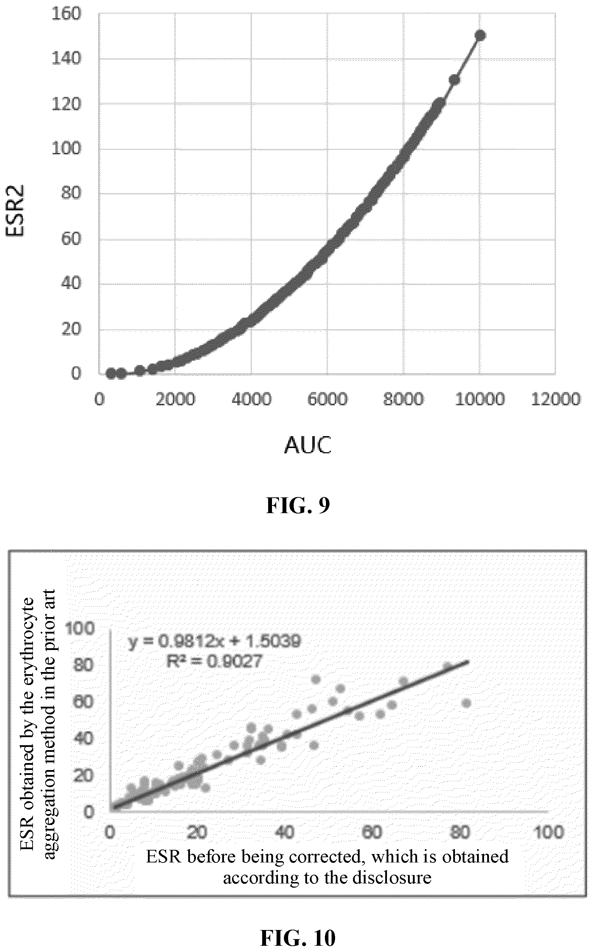

FIG. 9 shows a calibration curve (also referred to as a standard curve) for calculating the second ESR measurement result ESR2, where the abscissa thereof represents the AUC, and the ordinate thereof represents the second ESR measurement result ESR2. By researching, it is found that there is a correlation between the AUC of the erythrocyte aggregation curve and the ESR obtained by the Westergren method of the blood sample. Therefore, the calibration curve may be obtained by statistical fitting of the AUC of the erythrocyte aggregation curve and the ESR obtained by the Westergren method of a large number of blood samples. That is, a large number of blood samples are tested by using both the apparatus or system according to the disclosure and the measurement apparatus (including the ESR tube) for the Westergren method, to obtain the AUC of the erythrocyte aggregation curve and the ESR according to the Westergren method of these blood samples, and then a calibration curve may be obtained based on the data.

In some examples, the calibration curve is stored in the data processing devices 120 and 240 in the form of a fitting function or in the form of a series of discrete data. For example, the calibration curve is stored in the data processing devices 120 and 240 in the form of a lookup table, and the second ESR measurement result ESR2, which approximates to the ESR according to the Westergren method, may be obtained through the AUC by means of table lookup and interpolation.

The second ESR measurement result ESR2 obtained according to the example of the disclosure has a good correlation with a reference value for the erythrocyte aggregation method which is obtained by the instrument from ALIFAX company in the prior art, as shown in FIG. 10.

In some examples, the data processing devices 120 and 240 may be further configured to output at least one of the first ESR measurement result ESR1 and the second ESR measurement result ESR2.

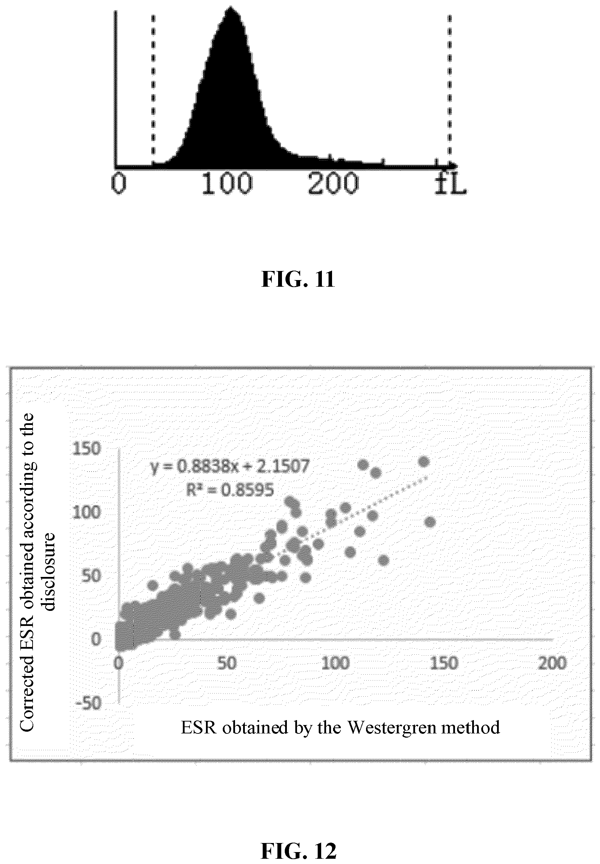

In some examples, the blood cell histogram may include one or more of a red blood cell volume distribution histogram, a platelet volume distribution histogram, and a white blood cell volume distribution histogram that are obtained based on a signal of impedance change detected by the impedance detection unit 232, as shown in FIG. 11. In a specific example, the erythrocyte aggregation curve may be corrected by using the red blood cell volume distribution histogram to obtain the ESR1. That is, the erythrocyte aggregation curve and the red blood cell volume distribution histogram of the blood sample to be tested are input into the pre-trained neural network model M to obtain an output of the neural network model as the ESR1.

In an example, the erythrocyte aggregation curve, a red blood cell volume distribution histogram obtained by the electrical impedance method, and a measurement result obtained by the Westergren method of a large number of blood samples are collected. Training is performed based on the neural network model M shown in FIG. 3 to obtain parameters of the neural network model. Next, the blood sample to be tested is detected by using the system 200 for measuring ESR according to the disclosure and the Westergren method, to obtain the corresponding first ESR measurement result ESR1 and the measurement result according to the Westergren method. The correlation shown in FIG. 12 is obtained based on the first ESR measurement result and the measurement result according to the Westergren method of the blood sample to be tested. It can be seen from FIG. 12 that the correlation between the first ESR measurement result ESR1 and the measurement result according to the Westergren method reaches 0.927, indicating that the first ESR measurement result ESR1 has a good consistency with the measurement result according to the Westergren method.

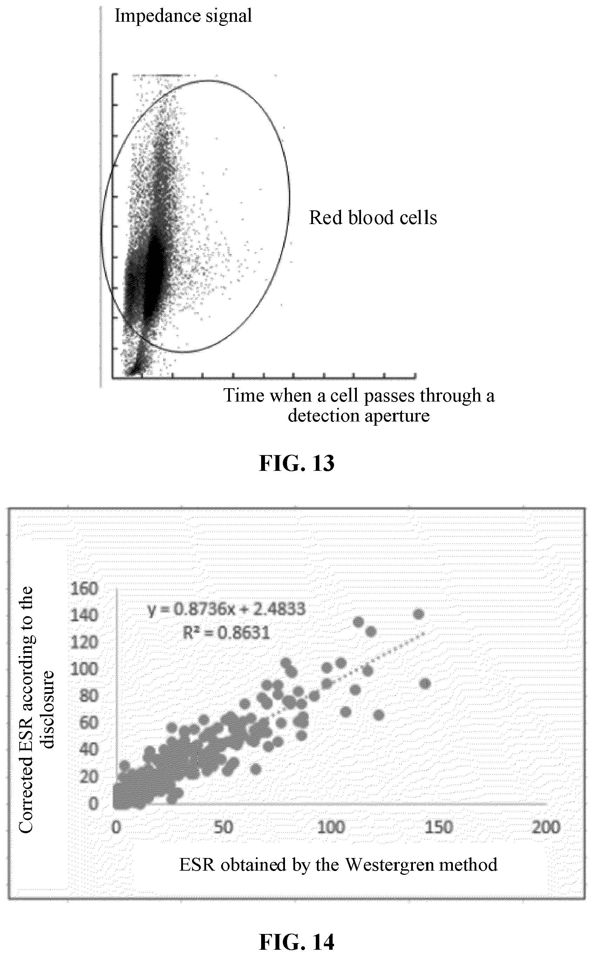

In some other examples, the blood cell scattergram may include one or more of a red blood cell scattergram, a platelet scattergram, and a white blood cell scattergram that are obtained based on a signal of impedance change detected by the impedance detection unit 232. For example, data for generating the red blood cell scattergram include volume signals of red blood cells, that is, signals of impedance change, and other signals related to the red blood cell passing through the detection aperture 2321, such as a time when the red blood cell passes through the detection aperture 2321 or a time when the red blood cell arrives at the center of the detection aperture 2321. As shown in FIG. 13, in the red blood cell scattergram shown therein, the abscissa thereof represents the time when the red blood cell passes through the detection aperture 2321, and the ordinate thereof represents an impedance signal.

In a specific example, the erythrocyte aggregation curve, a red blood cell scattergram obtained by the electrical impedance method, and a measurement result obtained by the Westergren method of a large number of blood samples are collected. Training is performed based on the neural network model M shown in FIG. 3 to obtain parameters of the neural network model. Next, the blood sample to be tested is detected by using the system 200 for measuring ESR according to the disclosure and the Westergren method to obtain the corresponding first ESR measurement result ESR1 and the measurement result according to the Westergren method. The correlation shown in FIG. 14 is obtained based on the first ESR measurement result and the measurement result according to the Westergren method of the blood sample to be tested. It can be seen from FIG. 14 that the correlation between the first ESR measurement result ESR1 and the measurement result according to the Westergren method reaches 0.929, indicating that the first ESR measurement result ESR1 has a good consistency with the measurement result according to the Westergren method.

Herein, it is advantageous to use a red blood cell volume distribution histogram, a platelet volume distribution histogram, a white blood cell volume distribution histogram, a red blood cell scattergram, a platelet scattergram, and/or a white blood cell scattergram that may be obtained based on the electrical impedance method, as the inputs to the neural network model, because in practice it is usually required for patients to detect a red blood cell parameter (red blood cell count) as well as a platelet parameter (platelet count) according to the electrical impedance method.

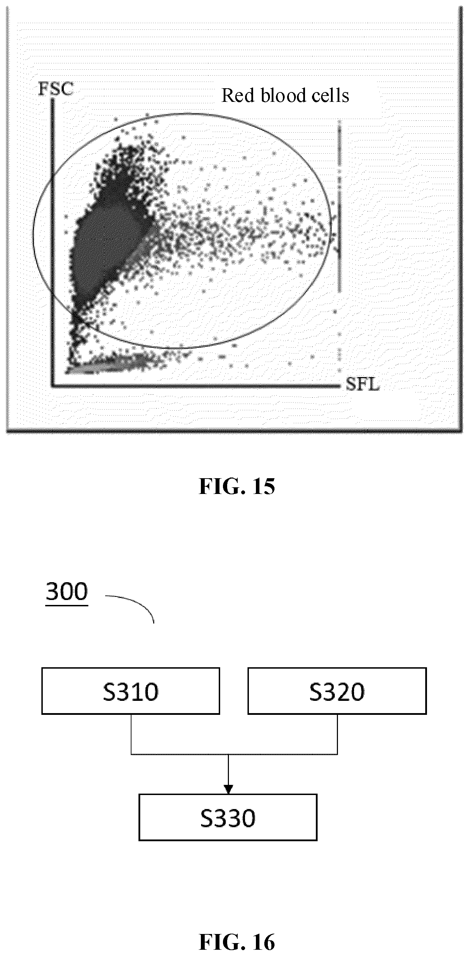

Alternatively, in still some other examples, the blood cell scattergram may include at least one of a red blood cell scattergram, a white blood cell scattergram, and a platelet scattergram that are obtained based on an optical signal detected by the optical detection unit 233. Preferably, the red blood cell scattergram obtained by using flow cytometry includes at least a forward-scattered light (FSC) signal of a red blood cell, and other signals forming the red blood cell scattergram may be a side-scattered light (SSC) signal and/or a side fluorescence (SFL) signal of a red blood cell. As shown in FIG. 15, in the red blood cell scattergram shown therein, the abscissa thereof represents the side fluorescence (SFL) signal of a red blood cell, and the ordinate thereof represents the forward-scattered light (FSC) signal of a red blood cell. For example, the erythrocyte aggregation curve of the blood sample to be tested and the red blood cell scattergram shown in FIG. 15 may be input into the pre-trained neural network model M to obtain an output of the neural network model as the ESR1.

In some examples, a blood cell histogram or a blood cell scattergram is used as an input parameter of the neural network model M. In some other examples, the blood cell histogram and the blood cell scattergram obtained in one test may be used as input parameters of the neural network model M.

As shown in FIG. 16, according to an example of the disclosure, a method 300 for measuring ESR is proposed, and the method 300 includes the following operations.

S310: an erythrocyte aggregation curve P1 of a blood sample to be tested is acquired.

S320: a blood cell histogram and/or a blood cell scattergram P2 of the blood sample to be tested are/is acquired, in which the blood cell histogram and/or the blood cell scattergram include/includes at least a histogram and/or a scattergram related to red blood cells.

S330: the erythrocyte aggregation curve and the blood cell histogram and/or the blood cell scattergram are input into a neural network model M, to calculate a first ESR measurement result ESR1 by using the neural network model M.

Further, the method may further include the following operations.

An AUC enclosed by the erythrocyte aggregation curve P1 and a time axis within a time period between a measurement start time point T1 and a measurement end time point T2 is calculated.

A second ESR measurement result ESR2 is calculated based on the AUC and a pre-stored calibration curve.

At least one of the first ESR measurement result ESR1 and the second ESR measurement result ESR2 is output.

In some examples, the blood cell histogram may include one or more of a red blood cell volume distribution histogram, a platelet volume distribution histogram, and a white blood cell volume distribution histogram obtained by using a Coulter method.

Additionally or alternatively, the blood cell scattergram may include a scattergram related to red blood cell volume measurement using the Coulter method and/or a scattergram related to red blood cell measurement using flow cytometry; and/or the blood cell scattergram may include a scattergram related to platelet volume measurement using the Coulter method and/or a scattergram related to platelet measurement using flow cytometry; and/or the blood cell scattergram may include a scattergram related to white blood cell measurement using flow cytometry.

In some examples, the erythrocyte aggregation curve may be obtained by means of transmission turbidimetry.

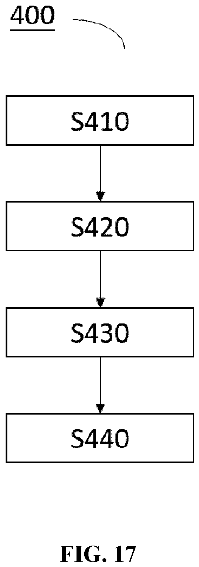

As shown in FIG. 17, according to another example of the disclosure, a method 400 for measuring ESR is proposed, and the method 400 includes the following operations.

S410: a portion of a blood sample to be tested is transferred to an optical testing pipeline 111, and red blood cells in the portion of the blood sample to be tested are de-aggregated by allowing the portion of the blood sample to be tested to flow back and forth in the optical testing pipeline 111.

S420: after de-aggregating the red blood cells, the portion of the blood sample to be tested is kept still in the optical testing pipeline 111, and is irradiated with light, to obtain an erythrocyte aggregation curve C of light intensity transmitted through the portion of the blood sample to be tested as a function of time.

S430: analysis data are input into a deep learning algorithm having a neural network structure, in which the analysis data include the erythrocyte aggregation curve and a blood cell histogram and/or a blood cell scattergram of the blood sample to be tested, and the blood cell histogram and/or the blood cell scattergram include/includes at least a histogram and/or a scattergram related to red blood cells.

S440: an ESR measurement result of the blood sample to be tested is calculated by means of the deep learning algorithm.

For example, the sampling needle of the system for measuring ESR aspirates the blood sample to be tested. After mixing homogenously, red blood cells in the blood sample to be tested are fully de-aggregated. A curve of light intensity transmitted through the de-aggregated blood sample as a function of time is measured, that is, the erythrocyte aggregation curve is measured.

For other examples and advantages of the method 300 and the method 400 for measuring ESR according to the disclosure, reference may be made to the above description of the apparatus 100 and the system 200 for measuring ESR according to the disclosure, and details will not be described herein again.

Further, an example not being part of the disclosure provides a computer-readable storage medium including computer program instructions. When the computer program instructions are executed by a processor, the operations of the above method 300 and of its various examples may be performed.

The features or combinations thereof mentioned in the above description, accompanying drawings, and claims can be used alone or in combination with each other arbitrarily, as long as it is within the scope of the appended claims and there is no conflict with each other. The advantages and features described for the apparatus and the system for measuring ESR provided in the disclosure are applicable in a corresponding manner to the method for measuring ESR provided in the disclosure, and vice versa.

The foregoing description merely illustrates the preferred examples of the disclosure, and is not intended to limit the scope of protection of the disclosure. The scope of protection of the disclosure shall be subject to the scope of protection of the claims.

## Claims

1. An apparatus (100) for measuring erythrocyte sedimentation rate, ESR, comprising: an ESR detection device (110) and a data processing device (120),
wherein the ESR detection device (110) is configured to test a blood sample to be tested, so as to obtain an erythrocyte aggregation curve of light intensity transmitted through or scattered by the blood sample to be tested as a function of time; and
the data processing device (120) is configured to acquire the erythrocyte aggregation curve of the blood sample to be tested;
**characterized in that** the data processing device (120) is further configured to: acquire a blood cell histogram and/or a blood cell scattergram of the blood sample to be tested which are/is obtained by a blood cell analyzer; and input the erythrocyte aggregation curve and the blood cell histogram and/or the blood cell scattergram into a neural network model (M), so as to calculate an ESR measurement result by using the neural network model (M).

2. The apparatus of claim 1, **characterized in that**, the ESR detection device (110) is further configured to: prior to obtaining the erythrocyte aggregation curve by means of testing, de-aggregate red blood cells in the blood sample to be tested by allowing the blood sample to be tested to flow back and forth in a testing region of the ESR detection device (110).

3. A system (200) for measuring erythrocyte sedimentation rate, ESR, comprising: a sampling and dispensing device (210), a blood cell detection device (230), and the apparatus (100) for measuring ESR of claim 1,
wherein the sampling and dispensing device (210) is configured to collect a blood sample to be tested, and dispense at least portions of the blood sample to be tested into the ESR detection device (110, 220) and the blood cell detection device (230) respectively;
the ESR detection device (110, 220) is configured to test the portion of the blood sample dispensed into the ESR detection device (110, 220), so as to obtain the erythrocyte aggregation curve of the light intensity transmitted through or scattered by the portion of the blood sample dispensed as a function of time;
the blood cell detection device (230) is configured to test the portion of the blood sample dispensed into the blood cell detection device (230), so as to obtain the blood cell histogram and/or the blood cell scattergram of the blood sample to be tested, the blood cell histogram and/or the blood cell scattergram comprising at least a histogram and/or a scattergram related to red blood cells; and
**characterized in that** the data processing device (120, 240) is configured to acquire the erythrocyte aggregation curve and the blood cell histogram and/or the blood cell scattergram, and input the erythrocyte aggregation curve and the blood cell histogram and/or the blood cell scattergram into the neural network model (M), so as to calculate a first ESR measurement result by using the neural network model (M).

4. The system of claim 3, **characterized in that**, the ESR detection device (110, 220) is further configured to: prior to obtaining the erythrocyte aggregation curve by means of testing, de-aggregate the red blood cells in the portion of the blood sample dispensed into the ESR detection device (110, 220) by allowing the portion of the blood sample dispensed to flow back and forth in a testing region of the ESR detection device (110, 220).

5. The system of claim 3 or 4, **characterized in that**, the data processing device (120, 240) is further configured to:
calculate an area enclosed by the erythrocyte aggregation curve and a time axis within a time period between a measurement start time point and a measurement end time point;
calculate a second ESR measurement result based on the area and a pre-stored calibration curve; and
output at least one of the first ESR measurement result and the second ESR measurement result.

6. The system of any of claims 3 to 5, **characterized in that**, the blood cell detection device (230) comprises an impedance detection unit (232) comprising a detection aperture (2321) and a detection circuit, the detection circuit being configured to detect a signal of impedance change when cells in the portion of the blood sample dispensed into the impedance detection unit (232) pass through the detection aperture (2321);
wherein the blood cell histogram comprises at least one of a red blood cell volume distribution histogram, a platelet volume distribution histogram, and a white blood cell volume distribution histogram that are obtained based on the signal of impedance change, and/or the blood cell scattergram comprises at least one of a red blood cell scattergram, a platelet scattergram, and a white blood cell scattergram that are obtained based on the signal of impedance change.

7. The system of any of claims 3 to 6, **characterized in that**, the blood cell detection device (230) comprises an optical detection unit (233) having a light source (101), a flow cell (103), and a light detector (104, 105, 107), wherein the light source (101) is configured to emit a light beam to irradiate a testing region of the flow cell (103), the light detector (104, 105, 107) is configured to detect an optical signal generated when cells in the portion of the blood sample dispensed into the optical detection unit (233) pass through the testing region and are irradiated with the light beam, and wherein the blood cell scattergram comprises at least one of a red blood cell scattergram, a white blood cell scattergram, and a platelet scattergram that are obtained based on the optical signal.

8. The system of any of claims 3 to 7, **characterized in that**, the neural network model (M) is pre-trained and stored in the data processing device (120, 240).

9. A method (300) for measuring erythrocyte sedimentation rate, ESR, comprising:
acquiring (S310) an erythrocyte aggregation curve of a blood sample to be tested;
acquiring (S320) a blood cell histogram and/or a blood cell scattergram of the blood sample to be tested, the blood cell histogram and/or the blood cell scattergram comprising at least a histogram and/or a scattergram related to red blood cells; and
**characterized in that** the method further comprises:
inputting (S330) the erythrocyte aggregation curve and the blood cell histogram and/or the blood cell scattergram into a neural network model (M), so as to calculate a first ESR measurement result by using the neural network model (M).

10. The method of claim 9, **characterized in that**, the method further comprises:
calculating an area enclosed by the erythrocyte aggregation curve and a time axis within a time period between a measurement start time point and a measurement end time point;
calculating a second ESR measurement result based on the area and a pre-stored calibration curve; and
outputting at least one of the first ESR measurement result and the second ESR measurement result.

11. The method of claim 9 or 10, **characterized in that**, the blood cell histogram comprises at least one of a red blood cell volume distribution histogram, a platelet volume distribution histogram, and a white blood cell volume distribution histogram obtained by using a Coulter method.

12. The method of any of claims 9 to 11, **characterized in that**, the blood cell scattergram comprises a scattergram related to red blood cell volume measurement using the Coulter method and/or a scattergram related to red blood cell measurement using flow cytometry; and/or
the blood cell scattergram comprises a scattergram related to platelet volume measurement using the Coulter method and/or a scattergram related to platelet measurement using flow cytometry; and/or
the blood cell scattergram comprises a scattergram related to white blood cell volume measurement using the Coulter method and/or a scattergram related to white blood cell measurement using flow cytometry.

13. The method of any of claims 9 to 12, **characterized in that**, the erythrocyte aggregation curve is obtained by transmission turbidimetry.

14. The method of any of claims 9 to 13, **characterized in that** acquiring (S310) the erythrocyte aggregation curve of the blood sample to be tested comprises:
transferring (S410) a portion of the blood sample to be tested to an optical testing pipeline (111), and de-aggregating red blood cells in the portion of the blood sample to be tested by allowing the portion of the blood sample to be tested to flow back and forth in the optical testing pipeline (111); and
after de-aggregating the red blood cells, keeping (S420) the portion of the blood sample to be tested still in the optical testing pipeline (111), and irradiating the portion of the blood sample to be tested with light, so as to obtain the erythrocyte aggregation curve of light intensity transmitted through the portion of the blood sample to be tested as a function of time;
wherein calculating the first ESR measurement result by using the neural network model (M) comprises:
inputting (S430) analysis data into a deep learning algorithm having a neural network structure, the analysis data comprising the erythrocyte aggregation curve and the blood cell histogram and/or the blood cell scattergram of the blood sample to be tested; and
calculating (S440) the ESR measurement result of the blood sample to be tested by means of the deep learning algorithm.

## Patentansprüche

1. Vorrichtung (100) zur Messung der Senkungsgeschwindigkeit der Erythrozyten, ESR, die Folgendes umfasst: ein ESR-Nachweisgerät (110) und ein Datenverarbeitungsgerät (120),
wobei das ESR-Nachweisgerät (110) so konfiguriert ist, dass sie eine zu untersuchende Blutprobe testet, um einen Erythrozyten-Aggregationskurve der Lichtintensität zu erhalten, die durch die zu testende Blutprobe hindurchgeht oder von dieser gestreut wird, als eine Funktion der Zeit; und
das Datenverarbeitungsgerät (120) so konfiguriert ist, dass es die Erythrozyten-Aggregationskurve der zu untersuchenden Blutprobe erfasst:
**dadurch gekennzeichnet, dass** das Datenverarbeitungsgerät (120) ferner so konfiguriert ist, dass es: ein Blutzellenhistogramm und/oder ein Blutzellen-Scattergramm der zu untersuchenden Blutprobe erfasst, die durch ein Blutzellenanalysegerät erhalten wird/werden; und die Erythrozyten-Aggregationskurve und das Blutzellenhistogramm und/oder das Blutzellen-Scattergramm in ein neuronales Netzmodell (M) eingibt, um ein ESR-Messergebnis unter Verwendung des neuronalen Netzmodells (M) zu berechnen.

2. Vorrichtung nach Anspruch 1, **dadurch gekennzeichnet, dass** das ESR-Nachweisgerät (110) ferner so konfiguriert ist, dass es vor dem Erhalt der Erythrozyten-Aggregationskurve mithilfe eines Test, die roten Blutkörperchen in der zu untersuchenden Blutprobe zerlegt, indem es die zu untersuchende Blutprobe in einem Testbereich des ESR-Nachweisgeräts (110) hin und her fließen lässt.

3. System (200) zur Messung der Senkungsgeschwindigkeit der Erythrozyten, ESR, das Folgendes umfasst: ein Probenahme- und Abgabesystem (210), ein Blutzellnachweisgerät (230) und die Vorrichtung (100) zur Messung der ESR nach Anspruch 1,
wobei das Probenahme- und Abgabegerät (210) so konfiguriert ist, dass es eine zu untersuchende Blutprobe entnimmt und zumindest Teile der zu untersuchenden Blutprobe in das ESR-Nachweisgerät (110, 220) bzw. das Blutzellnachweisgerät (230) abgibt;
wobei das ESR-Nachweisgerät (110, 220) so konfiguriert ist, dass es den in das ESR-Nachweisgerät (110, 220) abgegebenen Teil der Blutprobe prüft, um die Erythrozyten-Aggregationskurve der Lichtintensität, die durch den Teil der abgegebenen Blutprobe hindurchgeht oder von diesem gestreut wird, in Abhängigkeit von der Zeit zu erhalten;
wobei das Blutzellnachweisgerät (230) so konfiguriert ist, dass es den Teil der Blutprobe, der in das Blutzellnachweisgerät (230) abgegeben wird, prüft, um das Blutzellenhistogramm zu und/oder das Blutzellen-Scattergramm der zu untersuchenden Blutprobe zu erhalten, wobei das Blutzellenhistogramm und/oder das Blutzellen-Scattergramm mindestens ein Histogramm und/oder ein Streudiagramm umfasst, das sich auf rote Blutzellen bezieht; und
**dadurch gekennzeichnet, dass** das Datenverarbeitungsgerät (120, 240) so konfiguriert ist, dass es die Erythrozytenaggregationskurve und das Blutzellenhistogramm und/oder das Blutzellen-Scatterdiagramm erfasst und die Erythrozytenaggregationskurve und das Blutzellenhistogramm und/oder das Blutzellen-Scattergramm in das Modell des neuronalen Netzes (M) eingibt, um ein erstes ESR-Messergebnis unter Verwendung des Modells des neuronalen Netzes (M) zu berechnen.

4. System nach Anspruch 3, **dadurch gekennzeichnet, dass** das ESR-Nachweisgerät (110, 220) ferner so konfiguriert ist, dass es vor dem Erhalt der Erythrozyten-Aggregationskurve durch Testen, die roten Blutkörperchen in dem Teil der Blutprobe zerlegt, der in das ESR-Nachweisgerät (110, 220) abgegeben wird, wobei es den Teil der abgegebenen Blutprobe in einem Testbereich des ESR-Nachweisgeräts (110, 220) hin- und her fließen lässt.

5. System nach Anspruch 3 oder 4, **dadurch gekennzeichnet, dass** das Datenverarbeitungsgerät (120, 240) ferner so konfiguriert ist, dass es:
Eine Fläche berechnet, die von der Erythrozytenaggregationskurve und einer Zeitachse innerhalb einer Zeitspanne zwischen einem Start- und einem Endzeitpunkt der Messung eingeschlossen wird;
Ein zweites ESR-Messergebnis auf der Grundlage der Fläche und einer vorab gespeicherten Kalibrierungskurve berechnet; und
Ausgabe mindestens eines der Ergebnisse der ersten ESR-Messung und der zweiten ESR-Messung.

6. System nach einem der Ansprüche 3 bis 5, **dadurch gekennzeichnet, dass** das Blutzellennachweisgerät (230) eine Einheit zur Impedanzerfassung (232) umfasst, die eine Erfassungsblende (2321) und eine Erfassungsschaltung beinhaltet, wobei die Erfassungsschaltung so konfiguriert ist, dass sie ein Signal einer Impedanzänderung nachweist, wenn Zellen in dem Teil der Blutprobe, der in die Einheit zur Impedanzerfassung (232) abgegeben wird, durch die Erfassungsblende (2321) hindurchgehen;
wobei das Blutzellenhistogramm mindestens eines der folgenden Histogramme umfasst: ein Volumenverteilungshistogramm der roten Blutkörperchen, ein Volumenverteilungshistogramm der Blutplättchen und ein 20 Volumenverteilungshistogramm der weißen Blutkörperchen, die entsprechend des Signals der Impedanzänderung erhalten werden, und/oder das Blutzellen-Scattergramm umfasst mindestens eines von einem Erythrozyten-Scattergramm, einem Thrombozyten-Scattergramm und einem Leukozyten-Scattergramm, die entsprechend des Signals der Impedanzänderung erhalten werden.

7. System nach einem der Ansprüche 3 bis 6, **dadurch gekennzeichnet, dass** das Blutzellennachweisgerät (230) eine optische Nachweiseinheit (233) mit einer Lichtquelle (101), einer Durchflusszelle (103) und einem Lichtdetektor (104, 105, 107) umfasst, wobei die Lichtquelle (101) so konfiguriert ist, dass sie einen Lichtstrahl aussendet, um einen Testbereich der Durchflusszelle (103) zu bestrahlen; der Lichtdetektor (104, 105, 107) konfiguriert ist, um ein optisches Signal zu ermitteln, das erzeugt wird, wenn Zellen in dem Teil der Blutprobe, der in die optische Nachweiseinheit (233) abgegeben wird, durch den Testbereich hindurchgehen und durch den Lichtstrahl bestrahlt werden, und wobei das Blutzellen-Scattergramm mindestens eines von einem Scattergramm der roten Blutkörperchen, einem Scattergramm der weißen Blutkörperchen und einem Scattergramm der Blutplättchen umfasst, die auf der Grundlage des optischen Signals erhalten werden.

8. System nach einem der Ansprüche 3 bis 7, **dadurch gekennzeichnet, dass** das Modell des neuronalen Netzes (M) vortrainiert und in der Datenverarbeitungseinheit (120, 240) gespeichert wird.

9. Verfahren (300) zur Messung der Senkungsgeschwindigkeit der Erythrozyten, ESR, das Folgendes umfasst:
Erfassen (S310) eines Erythrozyten-Aggregationskurve einer zu untersuchenden Blutprobe:
Erfassen (S320) eines Blutzellenhistogramms und/oder eines Blutzellen-Scattergramms der zu untersuchenden Blutprobe, wobei das Blutzellenhistogramm und/oder das Blutzellen-Scattergramm mindestens ein Histogramm und/oder ein Scattergramm umfasst, das sich auf rote Blutkörperchen bezieht; und **dadurch gekennzeichnet, dass** das Verfahren ferner umfasst:
Eingeben (S330) der Erythrozytenaggregationskurve und des Blutzellenhistogramms und/oder des Blutzellen-Scattergramms in ein Modell des neuronalen Netzes (M), um ein erstes ESR-Messungsergebnis unter Verwendung des Modells des neuronalen Netzes (M) zu berechnen.

10. Verfahren nach Anspruch 9, **dadurch gekennzeichnet, dass** das Verfahren ferner Folgendes
umfasst:
Berechnung einer Fläche, die von der Erythrozytenaggregationskurve und einer Zeitachse innerhalb einer Zeitspanne zwischen einem Start- und einem Endzeitpunkt der Messung eingeschlossen wird:
Berechnung eines zweiten ESR-Messergebnisses auf der Grundlage der Fläche und einer vorab gespeicherten Kalibrierungskurve; und
Ausgabe mindestens eines der Ergebnisse der ersten und der zweiten ESR-Messung.

11. Verfahren nach Anspruch 9 oder 10, **dadurch gekennzeichnet, dass** das Blutzellenhistogramm mindestens eines der folgenden Histogramme beinhaltet: ein Volumenverteilungshistogramm der roten Blutkörperchen, ein Volumenverteilungshistogramm der Blutplättchen und ein Volumenverteilungshistogramm der weißen Blutkörperchen, die unter Verwendung eines Coulter-Zählers erhalten wurden.

12. Verfahren nach einem der Ansprüche 9 bis 11, **dadurch gekennzeichnet, dass** das Blutzellen-Scattergramm ein Scattergramm beinhaltet, das sich auf die Messung des Volumens roter Blutkörperchen unter Verwendung des Coulter-Zählers und/oder ein Scattergramm umfasst, das sich auf die Messung roter Blutkörperchen unter Verwendung der Durchflusszytometrie bezieht; und/oder
das Blutzellen-Scattergramm ein Scattergramm beinhaltet, das sich auf die Messung des Thrombozytenvolumens unter Verwendung des Coulter-Zählers bezieht, und/oder ein Scattergramm, das sich auf die Messung der Thrombozyten unter Verwendung der Durchflusszytometrie bezieht; und/oder
das Blutzellen-Scattergramm ein Scattergramm beinhaltet, das sich auf die Messung des Volumens der weißen Blutkörperchen nach der Coulter-Methode bezieht, und/oder ein Scattergramm, das sich auf die Messung der weißen Blutkörperchen unter der Verwendung der Durchflusszytometrie bezieht.

13. Verfahren nach einem der Ansprüche 9 bis 12, **dadurch gekennzeichnet, dass** die Erythrozytenaggregationskurve durch Übertragungs-Turbidimetrie erhalten wird.

14. Verfahren nach einem der Ansprüche 9 bis 13, **dadurch gekennzeichnet, dass** die Erfassung (S310) der Erythrozyten-Aggregationskurve der zu untersuchenden Blutprobe Folgendes umfasst:
Überführen (S410) eines Teils der zu untersuchenden Blutprobe in eine optische Testpipeline (111) und Auflösen der roten Blutkörperchen in dem Teil der zu testenden Blutprobe, indem man den Teil der zu testenden Blutprobe in der optischen Testpipeline (111) hin
und her fließen lässt; und nach dem Zerlegen der roten Blutkörperchen den Teil der zu untersuchenden Blutprobe noch in der optischen Testpipeline (111) zu halten (S420) und den Teil der testenden Blutprobe mit Licht zu bestrahlen, um die Erythrozytenaggregationskurve der Lichtintensität zu erhalten, die durch den Teil der zu untersuchenden Blutprobe in Abhängigkeit von der Zeit übertragen wird;
wobei die Berechnung des ersten ESR-Messergebnisses unter Verwendung des neuronalen Netzmodells (M) Folgendes umfasst:
Eingeben (S430) von Analysedaten in einen Deep-Learning-Algorithmus mit einer neuronalen Netzstruktur, wobei die Analysedaten die Erythrozytenaggregationskurve und das Blutzellenhistogramm und/oder das Blutzellen-Scattergramm der zu testenden Blutprobe umfassen; und
Berechnung (S440) des ESR-Messergebnisses der zu untersuchenden Blutprobe mithilfe des Deep-Learning-Algorithmus.

## Revendications

1. Appareil (100) de mesure de la vitesse de sédimentation des érythrocytes, VSE, comprenant : un dispositif de détection de VSE (110) et un dispositif de traitement de données (120),
où le dispositif de détection de VSE (110) est configuré pour tester un échantillon de sang à tester, de sorte à obtenir une courbe d'agrégation d'érythrocytes d'intensité lumineuse transmise à travers ou diffusée par l'échantillon de sang à tester en fonction du temps ; et
le dispositif de traitement de données (120) est configuré pour acquérir la courbe d'agrégation d'érythrocytes de l'échantillon de sang à tester ;
**caractérisé en ce que** le dispositif de traitement de données (120) est en outre configuré pour : acquérir un histogramme de cellules sanguines et/ou un graphique en nuage de points de cellules sanguines de l'échantillon de sang à tester lequel/lesquels est/sont obtenus par un analyseur de cellules sanguines ; et entrer la courbe d'agrégation d'érythrocytes et l'histogramme de cellules sanguines et/ou le graphique en nuage de points de cellules sanguines dans un modèle de réseau neural (M), de sorte à calculer un résultat de mesure de VSE en utilisant le modèle de réseau neural (M).

2. Appareil selon la revendication 1, **caractérisé en ce que** le dispositif de détection de VSE (110) est en outre configuré pour : avant d'obtenir la courbe d'agrégation d'érythrocytes par test, désagréger des globules rouges dans l'échantillon de sang à tester en permettant à l'échantillon de sang à tester de s'écouler en va-et-vient dans une zone de test du dispositif de détection de VSE (110).

3. Système (200) de mesure de la vitesse de sédimentation des érythrocytes, VSE, comprenant : un dispositif d'échantillonnage et de distribution (210), un dispositif de détection de cellules sanguines (230), et l'appareil (100) de mesure de la VSE selon la revendication 1,
où le dispositif d'échantillonnage et de distribution (210) est configuré pour recueillir un échantillon de sang à tester, et distribuer au moins des parties de l'échantillon de sang à tester dans le dispositif de détection de VSE (110, 220) et le dispositif de détection de cellules sanguines (230) respectivement ;
le dispositif de détection de VSE (110, 220) est configuré pour tester la partie de l'échantillon de sang distribuée dans le dispositif de détection de VSE (110, 220), de sorte à obtenir la courbe d'agrégation d'érythrocytes de l'intensité lumineuse transmise à travers ou diffusée par la partie de l'échantillon de sang distribuée en fonction du temps ;
le dispositif de détection de cellules sanguines (230) est configuré pour tester la partie de l'échantillon de sang distribuée dans le dispositif de détection de cellules sanguines (230), de sorte à obtenir l'histogramme de cellules sanguines et/ou le graphique en nuage de points de cellules sanguines de l'échantillon de sang à tester, l'histogramme de cellules sanguines et/ou le graphique en nuage de points de cellules sanguines comprenant au moins un histogramme et/ou un graphique en nuage de points associés à des globules rouges ; et
**caractérisé en ce que** le dispositif de traitement de données (120, 240) est configuré pour la courbe d'agrégation d'érythrocytes et l'histogramme de cellules sanguines et/ou le graphique en nuage de points de cellules sanguines, et entrer la courbe d'agrégation d'érythrocytes et l'histogramme de cellules sanguines et/ou le graphique en nuage de points de cellules sanguines dans le modèle de réseau neural (M), de sorte à calculer un premier résultat de mesure de VSE en utilisant le modèle de réseau neural (M).

4. Système selon la revendication 3, **caractérisé en ce que**, le dispositif de détection de VSE (110, 220) est en outre configuré pour: avant d'obtenir la courbe d'agrégation d'érythrocytes par test, désagréger les globules rouges dans la partie de l'échantillon de sang distribuée dans le dispositif de détection de VSE (110, 220) en permettant à la partie de l'échantillon de sang distribuée de s'écouler en va-et-vient dans une zone de test du dispositif de détection de VSE (110, 220).

5. Système selon la revendication 3 ou 4, **caractérisé en ce que**, le dispositif de traitement de données (120, 240) est en outre configuré pour :
calculer une aire délimitée par la courbe d'agrégation d'érythrocytes et un axe de temps dans une période entre un instant de début de mesure et un instant de fin de mesure ;
calculer un second résultat de mesure de VSE sur la base de l'aire et d'une courbe d'étalonnage pré-mémorisée ; et
fournir en sortie au moins un résultat parmi le premier résultat de mesure de VSE et le second résultat de mesure de VSE.

6. Système selon l'une quelconque des revendications 3 à 5, **caractérisé en ce que**, le dispositif de détection de cellules sanguines (230) comprend une unité de détection d'impédance (232) comprenant une ouverture de détection (2321) et un circuit de détection, le circuit de détection étant configuré pour détecter un signal de variation d'impédance lorsque des cellules dans la partie de l'échantillon de sang distribuée dans l'unité de détection d'impédance (232) passent à travers l'ouverture de détection (2321) ;
où l'histogramme de cellules sanguines comprend au moins un histogramme parmi un histogramme de distribution de volume de globules rouges, un histogramme de distribution de volume de plaquettes, et un histogramme de distribution de volume de globules blancs qui sont obtenus sur la base du signal de variation d'impédance, et/ou le graphique en nuage de points de cellules sanguines comprend au moins un graphique en nuage de points parmi un graphique en nuage de points de globules rouges, un graphique en nuage de points de plaquettes, et un graphique en nuage de points de globules blancs qui sont obtenus sur la base du signal de variation d'impédance.

7. Système selon l'une quelconque des revendications 3 à 6, **caractérisé en ce que**, le dispositif de détection de cellules sanguines (230) comprend une unité de détection optique (233) présentant une source de lumière (101), une cellule d'écoulement (103), et un détecteur de lumière (104, 105, 107), où la source de lumière (101) est configurée pour émettre un faisceau lumineux pour irradier une zone de test de la cellule d'écoulement (103), le détecteur de lumière (104, 105, 107) est configuré pour détecter un signal optique généré lorsque des cellules dans la partie de l'échantillon de sang distribuée dans l'unité de détection optique (233) traversent la zone de test et sont irradiées par le faisceau lumineux, et où le graphique en nuage de points de cellules sanguines comprend au moins un graphique en nuage de points parmi un graphique en nuage de points de globules rouges, un graphique en nuage de points de globules blancs, et un graphique en nuage de points de plaquettes qui sont obtenus sur la base du signal optique.

8. Système selon l'une quelconque des revendications 3 à 7, **caractérisé en ce que**, le modèle de réseau neural (M) est pré-entraîné et mémorisé dans le dispositif de traitement de données (120, 240).

9. Procédé (300) de mesure de la vitesse de sédimentation des érythrocytes, VSE, comprenant les étapes consistant à :
acquérir (S310) une courbe d'agrégation d'érythrocytes d'un échantillon de sang à tester ;
acquérir (S320) un histogramme de cellules sanguines et/ou un graphique en nuage de points de cellules sanguines de l'échantillon de sang à tester, l'histogramme de cellules sanguines et/ou le graphique en nuage de points de cellules sanguines comprenant au moins un histogramme et/ou un graphique en nuage de points associés à des globules rouges ; et
**caractérisé en ce que** le procédé comprend en outre :
le fait d'entrer (S330) la courbe d'agrégation d'érythrocytes et l'histogramme de cellules sanguines et/ou le graphique en nuage de points de cellules sanguines dans un modèle de réseau neural (M), de sorte à calculer un premier résultat de mesure de VSE en utilisant le modèle de réseau neural (M).

10. Procédé selon la revendication 9, **caractérisé en ce que**, le procédé comprend en outre :
le fait de calculer une aire délimitée par la courbe d'agrégation d'érythrocytes et un axe de temps dans une période entre un instant de début de mesure et un instant de fin de mesure ;
le fait de calculer un second résultat de mesure de VSE sur la base de l'aire et d'une courbe d'étalonnage pré-mémorisée ; et
le fait de fournir en sortie au moins un résultat parmi le premier résultat de mesure de VSE et le second résultat de mesure de VSE.

11. Procédé selon la revendication 9 ou 10, **caractérisé en ce que**, l'histogramme de cellules sanguines comprend au moins un histogramme parmi un histogramme de distribution de volume de globules rouges, un histogramme de distribution de volume de plaquettes, et un histogramme de distribution de volume de globules blancs obtenus en utilisant un procédé de Coulter.

12. Procédé selon l'une quelconque des revendications 9 à 11, **caractérisé en ce que**, le graphique en nuage de points de cellules sanguines comprend un graphique en nuage de points associé à une mesure de volume de globules rouges en utilisant le procédé de Coulter et/ou un graphique en nuage de points associé à une mesure de globules blancs en utilisant une cytométrie de flux ; et/ou
le graphique en nuage de points de cellules sanguines comprend un graphique en nuage de points associé à une mesure de volume de plaquettes en utilisant le procédé de Coulter et/ou un graphique en nuage de points associé à une mesure de plaquettes en utilisant une cytométrie de flux ; et/ou
le graphique en nuage de points de cellules sanguines comprend un graphique en nuage de points associé à une mesure de volume de globules blancs en utilisant le procédé de Coulter et/ou un graphique en nuage de points associé à une mesure de globules blancs en utilisant une cytométrie de flux.

13. Procédé selon l'une quelconque des revendications 9 à 12, **caractérisé en ce que**, la courbe d'agrégation d'érythrocytes est obtenue par turbidimétrie de transmission.

14. Procédé selon l'une quelconque des revendications 9 à 13, **caractérisé en ce que** le fait d'acquérir (S310) la courbe d'agrégation d'érythrocytes de l'échantillon de sang à tester comprend :
le fait de transférer (S410) une partie de l'échantillon de sang à tester vers une conduite de test optique (111), et de désagréger des globules rouges dans la partie de l'échantillon de sang à tester en permettant à la partie de l'échantillon de sang à tester de s'écouler en va-et-vient dans la conduite de test optique (111) ; et
après avoir désagréger les globules rouges, le fait de conserver (S420) la partie de l'échantillon de sang à tester dans la conduite de test optique (111), et d'irradier la partie de l'échantillon de sang à tester par de la lumière, de sorte à obtenir la courbe d'agrégation d'érythrocytes d'intensité lumineuse transmise à travers la partie de l'échantillon de sang à tester en fonction du temps ;
où le fait de calculer le premier résultat de mesure de VSE en utilisant le modèle de réseau neural (M) comprend :
le fait d'entrer (S430) des données d'analyse dans un algorithme d'apprentissage profond présentant une structure de réseau neural, les données d'analyse comprenant la courbe d'agrégation d'érythrocytes et l'histogramme de cellules sanguines et/ou le graphique en nuage de points de cellules sanguines de l'échantillon de sang à tester ; et
le fait de calculer (S440) le résultat de mesure de VSE de l'échantillon de sang à tester au moyen de l'algorithme d'apprentissage profond.
